# EUROPEAN PATENT APPLICATION

(11) **EP 4 755 348 A2**
(43) Date of publication of application: **10.06.2026**
(21) Application number: 26162902.6
(22) Date of filing: 22.06.2020
(51) Int. Cl.: A61F 2/24

(54) **ANNULOPLASTY MANUAL INTERNAL GUIDEWIRE NAVIGATION**

(30) Priority: 28.06.2019 US 201962868169 P
(62) Divisional of application: 20824713.0
(71) Applicant: Boston Scientific Scimed, Inc., Maple Grove, Minnesota 55311 (US)
(72) Inventor: ROHL, James P., Prescott, 54021 (US); SHUEY, Daniel, Pine City, 55063 (US); BALDWIN, Katherine L., Minneapolis, 55413 (US); ABBOTT, Aaron, Columbia Heights, 55421 (US); EGGERT, Joel T., Plymouth, 55442 (US); CAWTHRA, James K., Jr., Ramsey, 55303 (US); KILVINGTON, Jason A., Shoreview, 55126 (US); IHLBERG, Leo, Brookline, 02446 (US)
(74) Representative: Peterreins Schley

(57) **Abstract**

A minimally invasive endoluminal annuloplasty system and method of use includes a guide catheter configured to direct a working catheter to a treatment site within a cardiac cavity. The guide catheter may be translatably disposed within the working catheter which may be translatably disposed within an introducer sheath that extends into the cardiac cavity. The guide catheter may be sized to reduce noise, and/or may be formed from a material that assists with visualization. In one embodiment the guide catheter comprises a distal guidewire anchor. Visualization may be used to embed the distal guidewire anchor into tissue at the target site.

## Description

### FIELD

The present disclosure relates generally to the field of medical devices and more particularly to an annuloplasty system and method of use.

### BACKGROUND

The tricuspid valve lies between the right atrium and the right ventricle of the heart and functions to prevent back flow of blood from the right ventricle into the right atrium. The tricuspid valve is comprised of three leaflets, an anterior leaflet, a posterior leaflet, and a septal leaflet. Each leaflet is connected via chordae tendineae to anterior, posterior, and septal papillary muscles of the right ventricle, respectively.

In a healthy heart, the tricuspid valve functions as a one-way valve that closes at ventricular systole to prevent tricuspid regurgitation (TR) of blood into the right atrium. In a diseased heart, tricuspid dilation may cause the valve leaflets to no longer effectively close, or coapt, during systolic contraction. Consequently, regurgitation of blood occurs during ventricular contraction and cardiac output decreases.

The goal of tricuspid repair is to regain valve competence by restoring the physiological form and function of the valve apparatus. One exemplary technique may reduce the valve between the midpoint of the anterior leaflet and the coaptation point to the septum (CS) through clips or other means. Valve repair/replacement may be performed as an open-heart surgery or via endoluminal methods. In either case, arterial Fibrillation (AF) is a common side effect caused at least in part by inadvertent contact with a heart feature, and the associated damage to the cardiac nervous system. Inadvertent contact may result as valve repair components are delivered and positioned about the diseased valve by an annuloplasty catheter. In order to minimize inadvertent contact, positioning of the annuloplasty catheter may be visualized using echocardiography or the like. In some systems, the size of the annuloplasty catheter interferes with its visualization, impairing accuracy and increasing inadvertent contact with, and associated damage to, heart features. Positioning annuloplasty catheters may be particularly challenging for patients with pacemaker and defibrillator leads. Tricuspid valve tethering, the use of antiplatelet and anticoagulant medications, and the high TR recurrence rate may result from or be exacerbated by nervous system damage. It would be desirable to identify a valve repair system that reduces or overcomes these problems.

### SUMMARY

Embodiments of the present disclosure relate to a minimally invasive endoluminal annuloplasty system and method of use that includes a guide catheter configured to direct a working catheter to a treatment site within a heart. The guide catheter may be translatably disposed within the working catheter which may be translatably disposed within an introducer sheath configured to extend transluminally into the heart. The guide catheter may be sized for low noise, and/or may be formed from a material that aids visualization. In one embodiment the guide catheter comprises a distal guidewire anchor configured to embed into tissue at the treatment site. The working catheter may then be directly advanced over the guide catheter to the treatment site, thereby reducing the potential of inadvertent contact between the working catheter and other heart features.

According to one aspect, a system comprises an introducer sheath having a lumen extending from a proximal end to a distal end and configured to be transluminally advanced into a cardiac cavity including a treatment site, a working catheter configured for longitudinal translation within the introducer sheath to position a distal tip of the working catheter proximate to the distal end of the introducer sheath and a guide catheter translationally disposed within the working catheter, the guide catheter comprising a translation mechanism configured to advance a distal end of the guide catheter through the distal end of the working catheter to the treatment site to provide a path to the treatment site for the working catheter.

In some embodiments, the guide catheter may comprise a distal guidewire anchor disposed on the distal end, and the translation mechanism is configured to drive the distal guidewire anchor into tissue of the treatment site to secure the guide catheter to the treatment site. In various embodiments, the distal guidewire anchor may include at least one of a helical member, a clamp member, a hook member, or a needle member. A proximal end of the introducer sheath may comprise a steering controller coupled to the distal end of the introducer sheath to guide the distal end of the introducer sheath to the cardiac cavity. The working catheter may comprise a distal tissue engagement mechanism and a proximal tissue engagement control mechanism.

In some embodiments, the system may include an implant stylet, slidably disposable within a working channel of the working catheter, the implant stylet comprising at least one implant component supported within a distal end of the implant stylet. The system may include a staging structure comprising at least one of an introducer sheath support, a working catheter support or an implant stylet support. The staging structure may include a catheter stand comprising a rail, and the working catheter support may be slideably disposed on the rail of the catheter stand such that movement of the working catheter support along the rail translates the working catheter within the introducer sheath.

In some embodiments, the translation mechanism may be further configured to retract the guide catheter into the distal end of the working catheter. Some embodiments may include a visualization mechanism disposed proximate to the distal guidewire anchor upon one of the guide catheter, the distal guidewire anchor or the working catheter. The visualization mechanism may comprise, for example, an Intra-Cardiac Echo (ICE) transducer.

According to another aspect, an annuloplasty method includes the steps of transluminally advancing an introducer sheath having a lumen extending from a proximal end to a distal end into a cardiac cavity including a treatment site, advancing a distal end of a working catheter to the distal end of the introducer sheath, advancing a guide catheter past the distal end of the working catheter to the treatment site to provide a path for the working catheter and anchoring the distal end of the guide catheter to the treatment site.

In some embodiments, the method may include translating the working catheter to the treatment site along the path provided by the guide catheter. The method may further include withdrawing the working catheter from the treatments site over the guide catheter to position the distal end of the working catheter proximate to the distal end of the introducer sheath. The method may include releasing the distal end of the guide catheter from the treatment site, advancing the distal end of the guide catheter to a second treatment site, and advancing the distal end of the working catheter to the second treatment site over the guide catheter.

According to a further aspect, a catheter system includes a working catheter configured for advancement within an introduction catheter to at least a position within a cardiac cavity where a distal tip of the working catheter is removed from a treatment site of the cardiac cavity, a guide catheter translationally disposed within the working catheter, the guide catheter comprising a translation mechanism configured to advance the guide catheter through the working catheter to the treatment site, the guide catheter comprising an attachment mechanism for coupling a distal end of the guide catheter to the treatment site and a mechanism configured to slidably advance the working catheter along the guide catheter to the treatment site. In some embodiments, the catheter system may further include a distal guidewire anchor coupled to the guide catheter and the translation mechanism is configured to drive the distal guidewire anchor into tissue of the treatment site to secure the guide catheter to the treatment site. In various embodiments, the distal guidewire anchor may include at least one of a helical member, a clamp member, a hook member, or a needle member. The working catheter may comprise an annuloplasty catheter including a tissue engagement mechanism at its distal end including at least one of a needle, a hook, a clamp, a screw, a frame or an anchor. In some embodiments, the catheter system may include a visualization mechanism disposed proximate to the attachment mechanism of the guide catheter.

### BRIEF DESCRIPTION OF THE DRAWINGS

Non-limiting embodiments of the present disclosure are described by way of example with reference to the accompanying figures, which are schematic and not intended to be drawn to scale. In the figures, each identical or nearly identical component illustrated is typically represented by a single numeral. For purposes of clarity, not every component is labeled in every figure, nor is every component of each embodiment shown where illustration is not necessary to allow those of ordinary skill in the art to understand the disclosure. In the figures:
FIG. 1 illustrates an annuloplasty system in accordance with an embodiment of the present disclosure;
FIGS. 2A and 2B illustrate perspective views of an introducer sheath in accordance with one embodiment the present disclosure;
FIG. 3 is an alternate view of the annuloplasty system of FIG. 1;
FIGS. 4A, 4B, and 4C illustrate perspective views of a working catheter in accordance with one embodiment of the present disclosure;
FIGS. 5A and 5B illustrate perspective views of one embodiment of a distal end of a working catheter in accordance with the present disclosure;
FIG. 6 illustrates one embodiment of a proximal handle of a working catheter in accordance with the present disclosure;
FIG. 7 illustrates one embodiment of an introducer sheath in accordance with the present disclosure;
FIG. 8 illustrates one embodiment of a working catheter in accordance with the present disclosure;
FIGS. 9A and 9B illustrate embodiments of a guide catheter engaged at a treatment site as disclosed herein;
FIG. 10 illustrates an embodiment of a working catheter advanced along a guide catheter to a treatment site as disclosed herein;
FIG. 11 illustrates one embodiment of an annuloplasty system including an implant stylet in accordance with the present disclosure;
FIGS. 12A and 12B illustrate various views of one embodiment of a retracting distal end of a guidance catheter as disclosed herein;
FIGS. 13A-13C illustrate various embodiments of a visualization mechanism for the catheter system in accordance with the present disclosure;
FIGS. 14A and 14B illustrate one embodiment of an annuloplasty system including a tissue engaging frame in accordance with the present disclosure; and
FIGS. 15A and 15B illustrate one embodiment of an annuloplasty system including an over the wire tissue engagement mechanism in accordance with the present disclosure.

### DETAILED DESCRIPTION

According to one aspect a minimally invasive endoluminal annuloplasty system and method of use includes a guide catheter configured to direct a working catheter to a treatment site within a cardiac cavity. With such an arrangement, the potential for unintended contact and associated cardiac damage may be reduced through controlled travel and accurate placement of the working catheter during the annuloplasty procedure.

In one embodiment, the guide catheter may be translatably disposed within the working catheter which in turn may be translatably disposed within an introducer sheath that extends into the cardiac cavity. The guide catheter may be configured to facilitate visualization of its distal end. For example, the guide catheter may be sized to reduce noise, and/or may be formed from a material that assists with visualization. In one embodiment the guide catheter comprises a distal guidewire anchor. Visualization may be used to embed the distal guidewire anchor into tissue at the target site. The working catheter may then be advanced over the guide catheter directly to the treatment site with reduced potential of inadvertent contact with heart features. In some embodiments, visualization may be further improved by providing a channel for an intravascular cardiac echography (ICE) catheter within the working catheter. In some embodiments, the ICE catheter may be formed on a distal end of the guide catheter.

In some embodiments the annuloplasty system may further comprise an implant deployment system. The implant deployment system may include tissue engagement mechanisms and/or tissue reshaping mechanisms. The implant deployment system may be part of the working catheter or may comprise additional components that may be delivered through the working catheter using an implant stylet or other form of catheter.

According to one aspect, the annuloplasty system may include a plurality of nested catheters that increase in length as they decrease in diameter. Such an arrangement allows for one or more working catheters and/or implant deployment systems (including for example frames, anchors, screws, baskets, valves, clips and the like) to be interchangeably and precisely deployed over the guide catheter to a treatment site with minimal disruption.

These and other beneficial aspects of an annuloplasty system are described below. It should be noted that although embodiments of the present disclosure may be described with specific reference to tricuspid valves, the principles disclosed herein may be readily adapted to facilitate reconstruction of any valve annulus, for example including a mitral valve annulus and/or may similarly benefit any other dilatation, valve incompetency, valve leakage and other similar heart failure conditions.

As used herein, the term "distal" refers to the end farthest away from the medical professional when introducing a medical device into a patient, while the term "proximal" refers to the end closest to the medical professional when introducing a medical device into a patient.

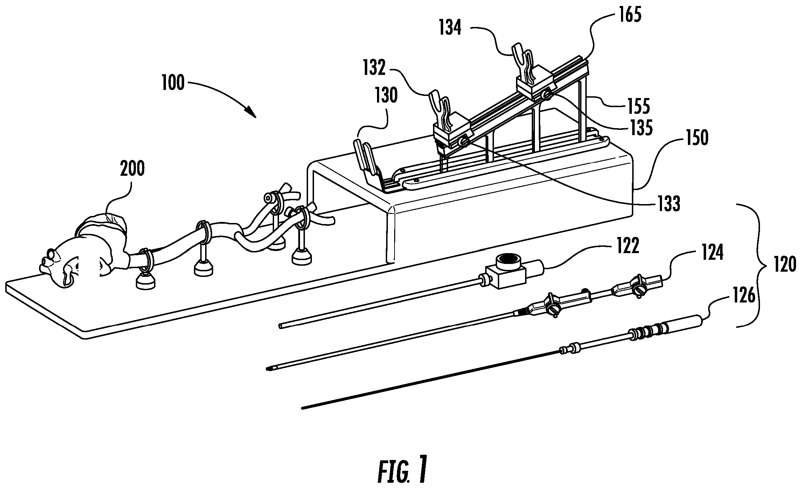

FIG. 1 illustrates a bench test setup including a representative heart 200 arranged for use with such an annuloplasty system 100. The annuloplasty system 100 is shown to comprise a nested catheter set 120 including a plurality of catheters which may be advanced individually or in combination transluminally into the heart 200 for repairing and/or replacing a valve or other heart feature. A stage 150 may be configured with a catheter stand 155 that may stabilize the annuloplasty system 100 during the annuloplasty procedure. Although FIG. 1 illustrates a bench set up, catheter stand 155 may be employed during an operative annuloplasty procedure to support the catheter set 120 as described below. In some embodiments, catheters of catheter set 120 may include proximal handles including one or more control mechanisms configured to control the movement and/or functionality of the catheter. The catheter stand 155 may comprise a plurality of handle mounts 130, 132, 134, each configured to support a handle of one of the catheters of the catheter set 120. In some embodiments, one or more of the handle mounts 130, 132, 134 may be slideably disposed upon a rail 165 of the catheter stand 155, and may be locked into place, for example by lockable brackets 133 or 135, when the distal ends of the catheters supported by the respective handles are positioned for annuloplasty.

In the embodiment of FIG. 1, the catheter set 120 is shown to include an introducer sheath 122, a working catheter 124 and an implant stylet 126. It should be understood that, although by way of example certain embodiments, number and arrangements of introducer, working and implant sheaths/catheters and/or stylets may be described, the principles disclosed herein may be extended to other types, number and arrangements of catheters.

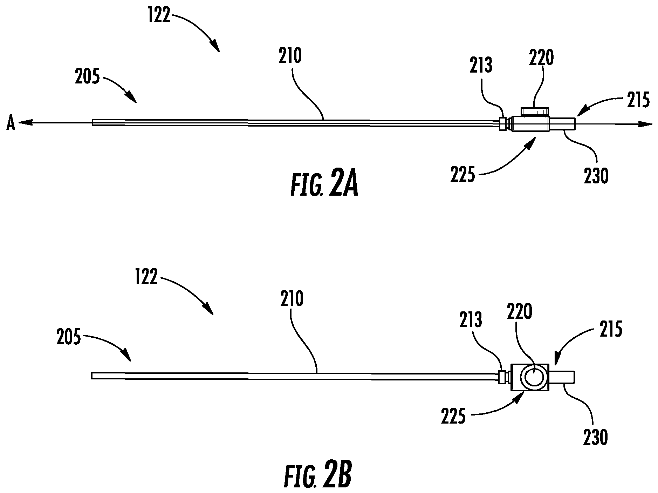

The introducer sheath 122 is shown from a side perspective in FIG. 2A and a top-down perspective in FIG. 2B. The introducer sheath 122 is shown to include a shaft 210 having a distal end 205 coupled to a handle 225 at the proximal end 215. The shaft 210 may be a steerable shaft comprising embedded pull cables which may be coupled to mechanisms in the handle 225 configured to deflect the distal end of the shaft 210 as it travels through the lumens of arteries or veins into the cardiac cavity. The handle 225 may include a steering control mechanism such as dial 220 which may control the deflection of the distal end 205 of the introducer sheath 122. Alternative steering control mechanisms may include, for example, thumbwheels, dials, knobs, switches and the like.

According to one aspect, the introducer sheath 122 includes an introducer lumen extending axially through catheter 122 as shown by line 'A' of FIG. 2A. The introducer lumen is sized to support working catheters, such as annuloplasty catheters including implant components for example.

In one embodiment, the introducer sheath 122 may comprise a composite of layers of thermoplastic elastomer (TPE), for example PEBAX provided by ARKEMA corporation of Colombes France. Alternatively, nylon, polyurethanes, polyester, silicone or other similar materials may be used to provide thin walls that may be extruded and layered over braided wires or coils for tensile and hoop strength, although the disclosed system is not limited to any particular material composition for the introducer sheath. In some embodiments, the length of the shaft 210 may range from between 24"-52", and more particularly between 42"-46". In one embodiment, the inner diameter may range between 24-31 Fr, and the outer diameter may range between 26Fr and 34Fr or more. In an exemplary embodiment, an inner diameter may be, for example 28Fr and the outer diameter may be 32Fr.

In one embodiment, a connector 230 may be disposed on the proximal end of the handle 225. The connector 230 may be configured for mated engagement with, for example, a working catheter. In some embodiments, the connector 230 may advantageously include a hemostasis valve to minimize blood loss during the annuloplasty procedure.

In some embodiments, the handle 225 may comprise a feature configured to engage, lock or otherwise coupled the handle 225 to the catheter stand 155 (FIG. 1) to minimize movement of the introducer sheath following placement. For example, introducer sheath 122 includes a neck 213, configured to engage with a quick release latch configuration of handle mount 130 (FIG. 1).

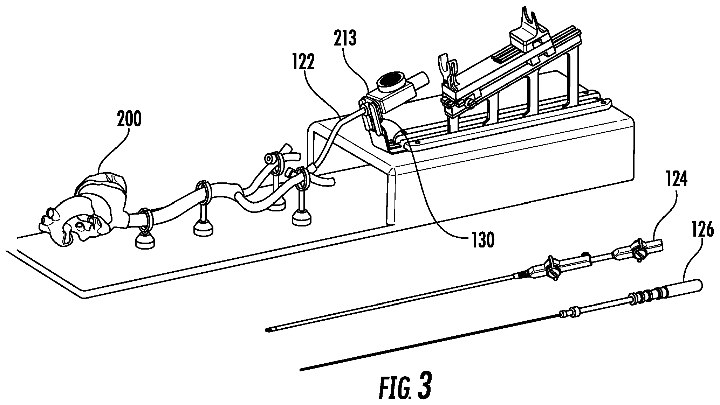

FIG. 3 illustrates an embodiment of the annuloplasty system arranged for testing on a test bed wherein a distal end of the introducer sheath 122 has been extended into a cavity of the heart 200. Neck 213 is shown locked into the quick release latch of handle mount 130 as during an annuloplasty procedure. Once the introducer is placed, the annuloplasty procedure may be initiated using, for example, the working catheter 124 and/or the stylet 126.

For the purposes of this application, a 'working' catheter may be any catheter carrying or controlling one or more components that may be used or configured to repair and/or replace a cardiac valve. Similar to the introducer sheath, the working catheter may be comprised of PEBAX, nylon, polyurethanes, polyester, silicone or other similar material. The working catheter may include one or more tissue engaging mechanisms at its distal end, for example needles, hooks, anchors, clips, screws, struts or the like, where the tissue engagement mechanisms may be configured to engage with cardiac tissue as part of the annuloplasty procedure. According to one aspect, the working catheter may also include (or be adapted to receive), a guide catheter, where the guide catheter guides the working catheter in a precise and controlled manner to a treatment site within the cardiac cavity as described below. Typically, a working catheter may range from between 6 Fr - 27 Fr. For example, given an introducer sheath having a 34 Fr OD and a 30 Fr ID, a 27 Fr or 28 Fr working catheter may translate within the introducer without restriction in a tight bend radius.

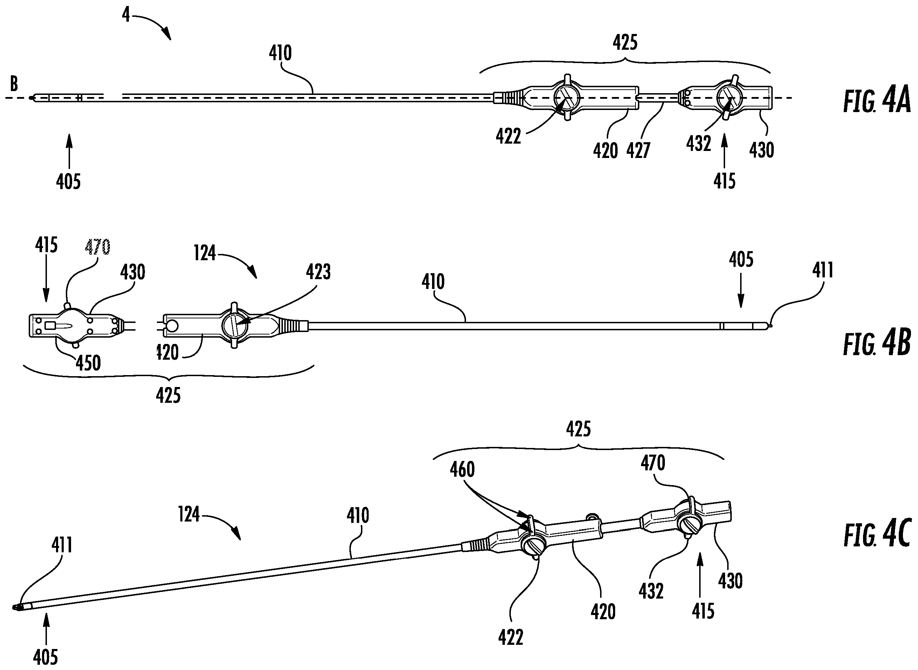

FIGS. 4A-4C illustrate an exemplary working catheter 124 in a number of perspective views. In FIG. 4A, a top-down perspective of working catheter 124 is shown to include a distal end 405, a proximal end 415, and a working shaft 410 disposed therebetween. A channel B extends between the proximal end 415 and distal end 405 of the working catheter 124. The proximal end 415 is shown to include a handle 425 comprising tissue engagement control portion 420 and a guide catheter control portion 430. The tissue engagement control portion 420 is shown to include a tissue engagement lock 422. In one embodiment, the tissue engagement lock 422 includes a dial which in a first position enables steering of the distal end 405 of the shaft 410, and in a second position locks the distal end of 405 of the shaft 410 in a fixed position. The guide catheter control portion 430 is shown in FIG. 4A to include a proximal end of guide catheter 427, disposed within channel B of the shaft 410, and a guide catheter lock 432. In one embodiment, in a first position the guide catheter lock 432 enables axial translation within channel B and steering of the guide catheter 427, and in a second position the guide catheter lock 432 locks the distal end of the guide catheter 427 in a fixed position.

In exemplary embodiments, the guide catheter 427 may be formed of material such as PEBAX, nylon, polyurethanes, polyester, silicone or other similar materials. In one embodiment, the guide catheter 427 is a steerable catheter measuring, for example, 9 Fr or less in diameter, slidably disposed at least within the distal end of the working catheter and configured to extend past a distal end of the shaft 410 of the working catheter 124. Due to its relatively small size, the guide catheter may be more easily visualized in the cardiac cavity, increasing the precision of placement of annuloplasty components during surgery.

FIG. 4B illustrates a bottom up perspective of a working catheter 124, showing the underside of the handle 425 at the proximal end 415, including guide catheter control portion 430 and tissue engagement control portion 420. In FIG. 4B, a distal guidewire anchor 411 is shown extending through a distal end 405 of the shaft 410 of the working catheter 124.

Guide catheter control portion 430 of the handle 425 is shown to include a steering controller 470 positioned on a first surface and a translation mechanism 450 disposed on a different surface. Although not a requirement, providing controls on different surfaces of the handle allows a surgeon to more easily steer and advance the distal end of the guide catheter with one hand. In one embodiment the steering controller 470 comprises a rotatable lever, although other similar mechanisms such as wheels, dials and the like may be substituted. The translation mechanism 450 may include a thumbwheel or other similar mechanism, wherein rotation of the thumbwheel in a first direction may advance the guide catheter through a distal end of the working catheter 124 and rotation of the thumbwheel in an opposite second direction may retract the guide catheter back into the working catheter 124.

FIG. 4C illustrates a side perspective of the working catheter 124, including the handle 425 having a tissue engagement control portion 420 and a guide catheter control portion 430 at a proximal end 415, a shaft 410 and a distal guidewire anchor 411 at a distal end 405. In FIG. 4C, the steering controller 470 can be seen in more detail to include a lever that may be controlled with a thumb or the like to steer the guide catheter while it is advanced or retracted by the translation mechanism 450 (FIG. 4B). Once the distal guidewire anchor 411 is secured, the guide catheter lock 432 may be activated to secure the position of the distal guidewire anchor 411. The tissue engagement control portion 420 may also include controls such as levers 460 that may be used to steer the distal end 405 of the shaft 410. Once the distal end 405 of the shaft 410 has been steered into a working position, the tissue engagement lock 422 may be activated to retain the working position of the shaft 410.

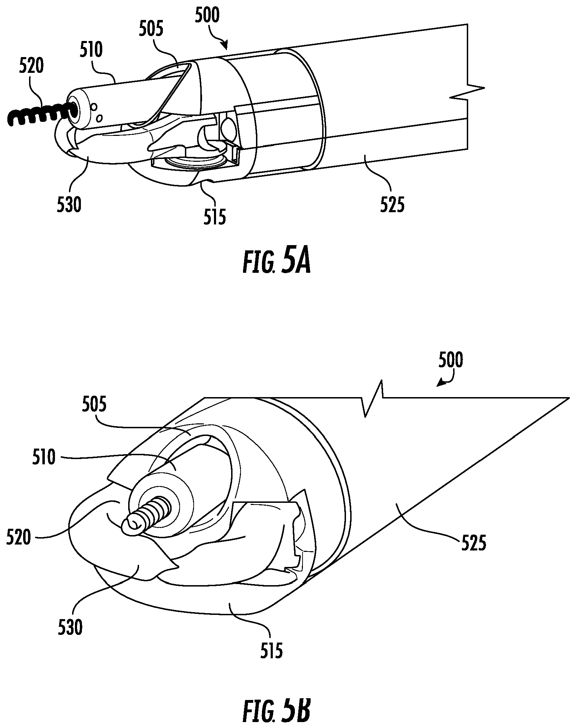

FIGS. 5A and 5B illustrate a distal end of a working catheter 500 in more detail, where FIG. 5A is a side view perspective of working catheter 500, and FIG. 5B is a proximally facing view of the distal end of catheter 500. In both figures, guide catheter 510 is shown extending through a channel 505 at the distal end of the working catheter 500. The working catheter 500 is also shown to include tissue engagement mechanisms, such as needles 530 which may be controlled by the tissue engagement mechanism features of the handle of the working catheter as described with regard to FIGS. 4A-4C. As mentioned previously, although needles are shown, FIGS. 5A and 5B illustrate only one form of working catheter, and other types of catheters including tissue engagement means such as clips, anchors, needles, struts, spurs, or similar mechanisms that may be used to at least temporarily engage the distal end of the working catheter to cardiac tissue for the annuloplasty procedure are considered equivalents.

In one embodiment the distal end of the guide catheter 510 may comprise a distal guidewire anchor 520. The distal guidewire anchor 520 may be comprised of stainless steel, nitinol, or the like, and may include a sharpened distal end configured to secure the guide catheter 510 to a treatment site. The distal guidewire anchor may range from about 2mm to 10 to about 15 millimeters (mm) in total axial length and from about 0.2 or less to 3 mm or more in diameter. The distal guidewire anchor 520 may comprise an attachment mechanism that may take many forms, including but not limited to a helix, a clamp, a hook, a needle or the like. In one embodiment, one or both of the distal guidewire anchor 520 and/or the distal portion of the guide catheter 510 may be comprised of materials such as nitinol, stainless steel, PEEK, polymer or other material that provide high visibility under echocardiography and/or fluoroscopy. In some embodiments, the distal guidewire anchor 520 may be provided with features that aid visualization, including but not limited to platinum Iridium marker bands (not shown) comprised of thin-walled seamless platinum-iridium alloy. The marker bands may also include radiopaque marking for catheter tubes and sensors during angioplasty and other procedures under fluoroscopy. Typical dimensions for marker bands include diameters from about 0.2-8.0 mm, wall thicknesses of about 0.015-0.5 mm, and lengths ranging from about 0.2 mm up to about 25 mm.

In one embodiment, a shaft 515 of the working catheter supports the tissue engagement mechanisms 530 and includes the working channel 505. In some embodiments, the working channel 505 supporting the guide catheter 510 may extend along at least a portion of the length of the working catheter 500. According to one aspect, the location of the working channel within the working catheter is selected so as to not interfere with the tissue engagement mechanisms 530 of the working catheter. A sheath 525 may be disposed over the shaft 515 and may be constructed of material such as PEBAX to facilitate smooth translation of the working catheter within the introducer sheath.

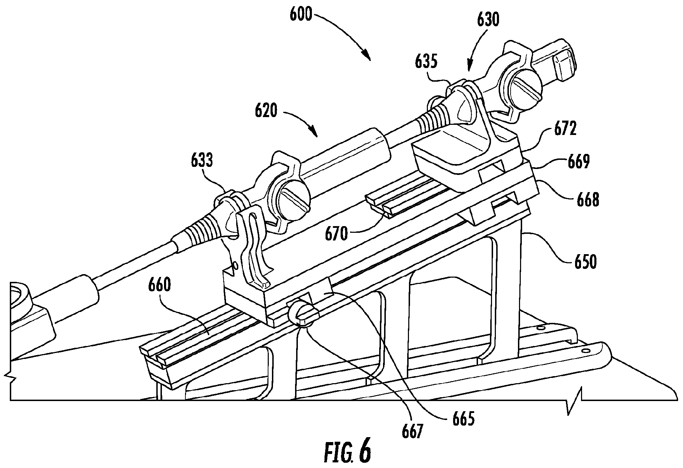

FIG. 6 illustrates a handle 600 of a working catheter secured on a catheter stand 650, wherein a bracket 633 is configured to support the tissue engagement portion 620 of the handle 600, and a bracket 635 is configured to support the guide catheter portion 630 of the handle 600. With such an arrangement, the working catheter can be secured against inadvertent movement of the handle 600 that may dislocate the distal end of the working catheter during use. In the illustrated embodiment, the catheter stand 650 is shown to comprise a rail 660 upon which a sled 669 is slideably disposed. In one embodiment, the sled 669 may include support members 665, 668 including ridges or other features configured to ride in the rail 660 to enable translation of the sled 669 along the rail. The position of the sled 668 on the rail 660 may be secured by a locking mechanism 667. In the embodiment of FIG. 6, a second rail 670 is shown disposed on the sled 669. A support member 672 includes features to slideably engage the support member 672 to the rail 670 and includes a bracket 635 configured to support the guide catheter portion 630 of the handle 600. Such an arrangement facilitates a controlled, manual introduction of the distal end of the guide catheter to an annuloplasty treatment site in a manner that minimizes the opportunity for inadvertent contact with heart features.

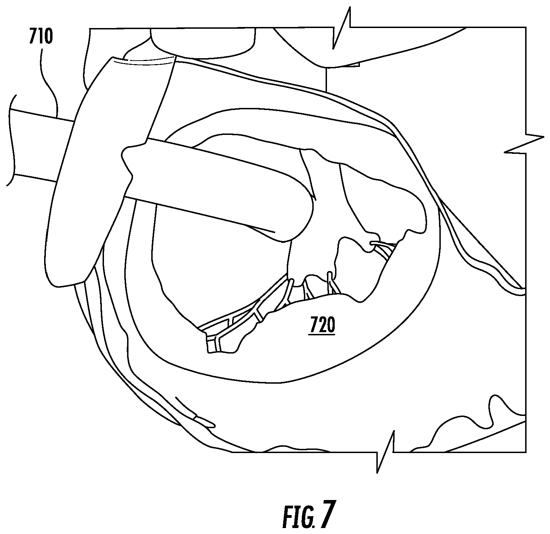

Referring now to FIGS. 7-13, one embodiment of a method of use of the annuloplasty catheter system disclosed herein is described. FIG. 7 is a top down view illustrating an introducer sheath 710 disposed in a staging position over a diseased valve 720. In one embodiment, a 'staging position' comprises a position wherein the distal end of the introducer sheath is advanced into a heart cavity but spaced away or otherwise removed from the treatment site. The spacing between the distal end of the introducer sheath 710 and the treatment site 720 may vary depending upon considerations including but not limited to patient anatomy, treatment site location, introducer sheath configuration, guide catheter length and available visualization techniques, but should be sufficient to enable visualization of the treatment site without interference by the introducer sheath or any catheters within the introducer sheath. For example, in one embodiment a staging position for an introducer sheath in an annuloplasty procedure may be a position wherein the introducer sheath is relatively centered above but spaced away from the valve or annulus, for example from between 1-2 mm and 35-55 mm away from the valve or annulus.

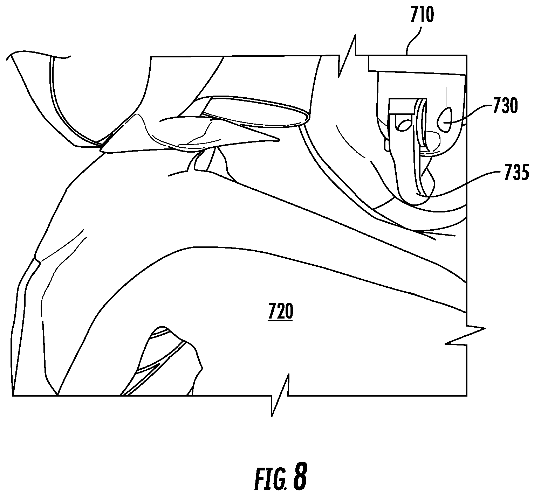

In FIG. 8, the working catheter 730 has been advanced through the introducer sheath 710 until the tissue engagement mechanism 735 at the distal end of the working catheter 730 is positioned proximate to the staging position of the introducer sheath 710. According to one embodiment, a staging position of a working catheter relates to the staging position of the introducer sheath and may comprise a position wherein the tissue engagement mechanisms 735 of the distal end of the working catheter 730 are exposed by the introducer sheath 710 but sufficiently removed from the valve 720 to enable visualization of the valve without interference from the working catheter 730.

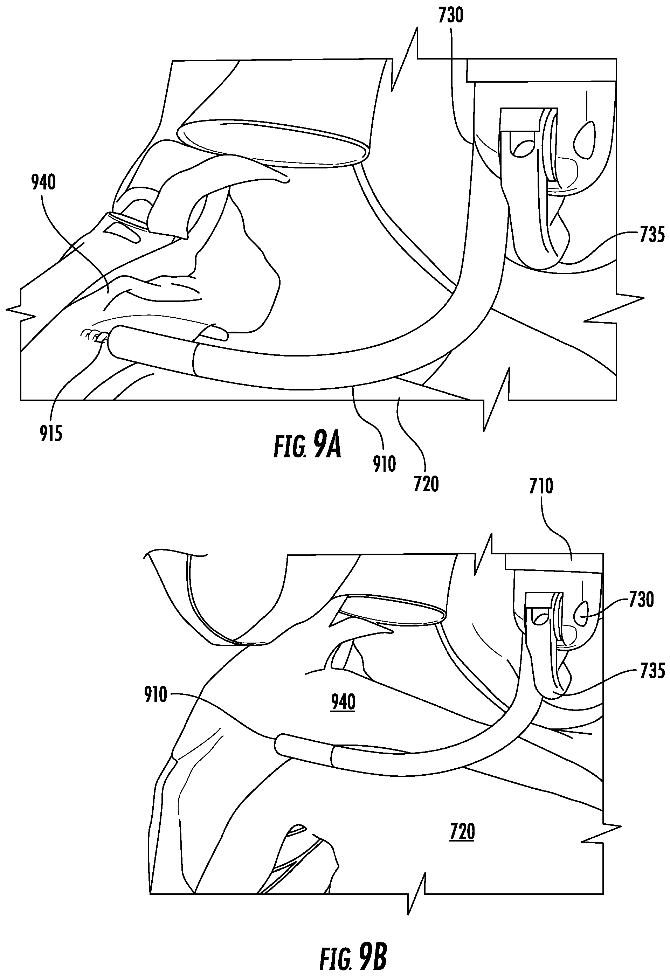

FIG. 9A illustrates the advancement of the guide catheter 910 from the working catheter 730 towards the wall 940 of the heart, while the tissue engagement mechanisms 735 of the working catheter 730 are held away from the valve in a working catheter staging position. As shown in FIG. 9A, the distal guidewire anchor 915, disposed at the distal end of guide catheter 910, may be advanced into the tissue until it is at least partially embedded. In FIG. 9B, the distal guidewire anchor 915 (not shown) is fully embedded within tissue of the cardiac wall 940 proximate to valve 720 and the guide catheter 910 provides a direct path to the cardiac wall 940. The working catheter 730 may then be advanced over the guide catheter 910 to bring the tissue engagement mechanisms 735 of the working catheter 730 to the cardiac wall 940 in a controlled manner.

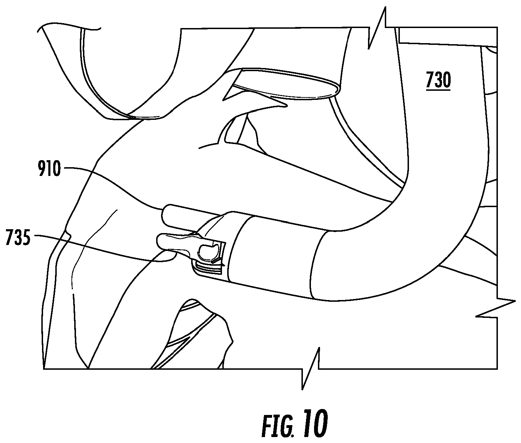

In FIG. 10, the working catheter 730 has been advanced along the path defined by the guide catheter 910, and tissue engagement mechanisms 735 are positioned next to the cardiac wall to begin the annuloplasty procedure. Using the guide catheter 910, which has a profile of 9Fr or less, to navigate to the treatment site greatly reduces the potential for inadvertent contact, and, in the event of contact, greatly reduces the impact caused by such contact. Once the guide catheter 910 has been deployed, thus precisely targeting the treatment site, the larger working catheter 730 may be deployed over the guide catheter in a controlled manner, thereby reducing adverse impact associated with annuloplasty system deployment. The working catheter 730 may be controlled to engage the cardiac tissue through manipulation of the tissue engagement mechanism controls on the handle of the working catheter. For example, in one embodiment, tissue engagement mechanisms 735 may be controlled to open and close to capture tissue of the cardiac wall, for example using the levers 460 (FIG. 4C) to open and close needles of the tissue engagement mechanism 735, and the lock 422 (FIG. 4A) to secure the needles in the tissue engaged orientation for delivery of annuloplasty repair components.

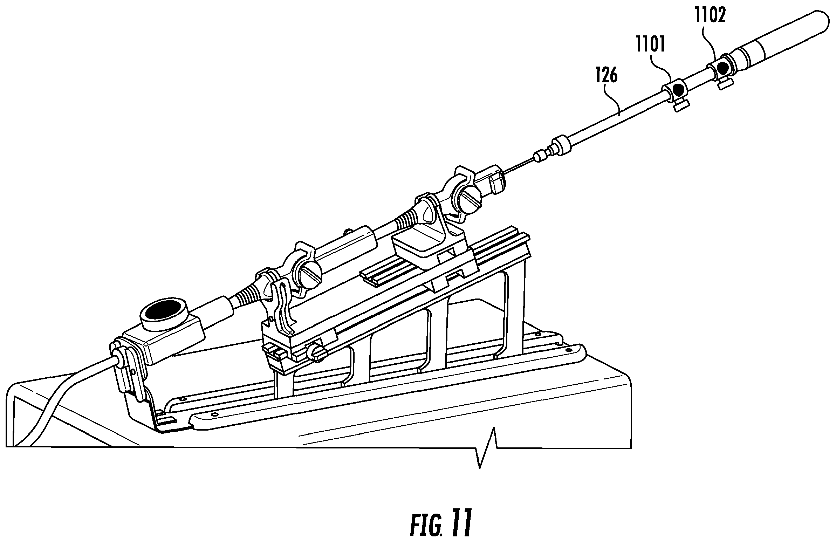

According to one aspect, as shown in FIG. 11 one or more annuloplasty components may be delivered to the cardiac cavity using an implant stylet 126. In one embodiment, the implant stylet 126 may extend through a lumen of the working catheter to its distal end, positioning the implant components precisely at the target treatment site. The construction of an implant stylet will vary by annuloplasty procedure. For example, the implant stylet may be configured to deploy one or more implant components including but not limited to a frame, a basket, a band, an anchor, a clip, a suture or other mechanism that may be used to reduce the size of a valve. Deployment of the implant components may be controlled in a variety of manners. For example, in FIG. 11, implant stylet is shown to include a plurality of collars 1101, 1102, each collar associated with an anchor. One or more implant components may be disposed at the distal end of the stylet. Advancement of the collars 1101, 1102 along the stylet may extrude or otherwise deploy the anchor through the distal end of the working catheter, for example by advancing a pusher mechanism or other such device to force the component from the tip. In some embodiments, a push button or dial may be used to deploy multiple components.

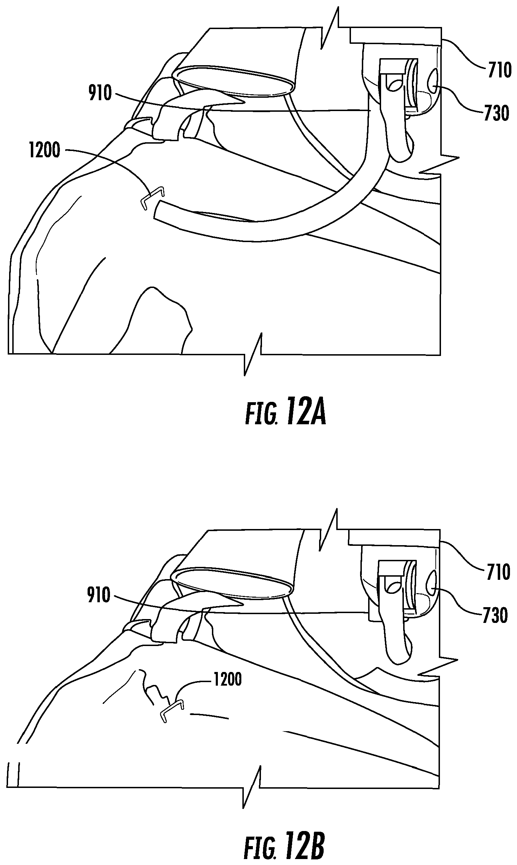

Following deployment and appropriate manipulation of the annuloplasty component, the working catheter 730 may be moved to the next treatment site within the cardiac cavity with minimal impact on heart features. Thus, in FIG. 12A, the working catheter 730 has been withdrawn back to the working catheter staging position as described above, where the distal end of the working catheter is proximate to but exposed from the distal end of the introducer sheath 710 in its staging position. Clip 1200 has been engaged to the target tissue site, and the guide catheter 910 may be released. In one embodiment, release may be achieved using the translation mechanism 450 (FIG. 4B), for example by rotating a thumbwheel to retract the distal guidewire anchor from the tissue. As shown in FIG. 4B, the guide catheter 910 may then be retracted into the working catheter 730 prior to being advanced to the next treatment site. In other embodiments, the guide catheter 910 may be moved directly to the next treatment site following release of the clip 1200 without being retracted into the working catheter 730. The disclosed annuloplasty system thus enables surgeons to flexibly modify an order of operations based on patient/diseased valve characteristics and personal preferences.

In some embodiments, the distal guidewire anchor may comprise a portion of the implant, and retraction of the guide catheter may include release of the distal guidewire anchor. In such embodiments, the guide catheter may comprise a plurality of distal guidewire anchors disposed in series within a lumen of the guide catheter, and extruded, for example by action of a pushbutton or other method, at each target treatment site.

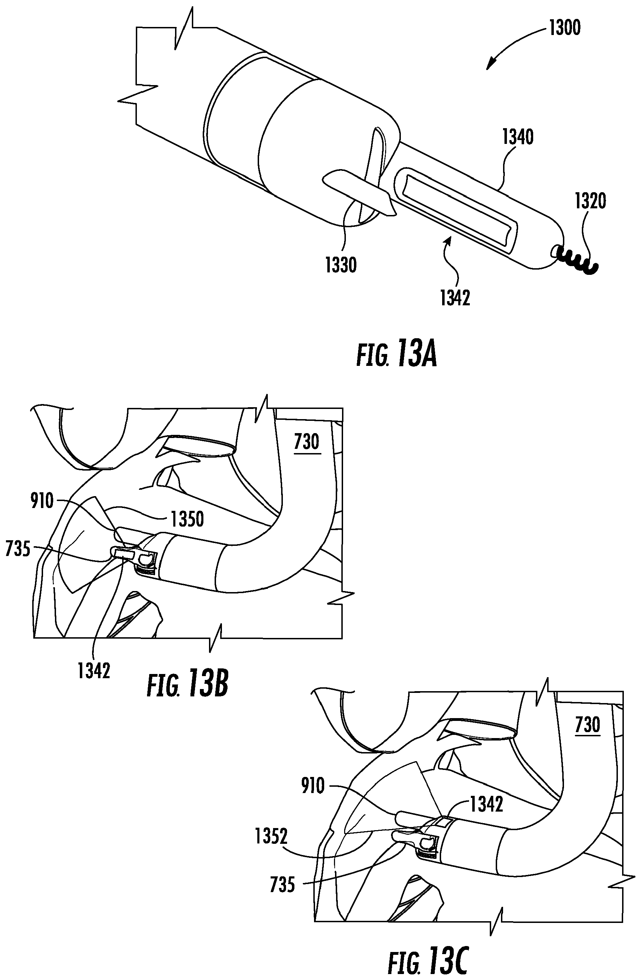

Accordingly, a system and method for valve repair and/or replacement with improved visualization and precision has been shown and described. According to one aspect, it is recognized that the benefits in precision may be further enhanced by including visualization means at the distal end of the guide catheter. FIG. 13A illustrates a distal end of an exemplary guide catheter 1300 including a distal guidewire anchor 1320 coupled to an intravascular cardiac echography (ICE) catheter 1340 comprising an ICE sensor array 1342. As discussed above, the distal end of the working catheter may also include tissue engagement mechanisms such as needle 1330. In one embodiment, the ICE catheter 1340 may be rotated within the lumen of the working catheter supporting the guide catheter to visualize various treatment sites within the cardiac cavity. In some embodiments, a guide catheter comprising ICE imaging comprises a profile that is 12Fr or less, thereby enhancing visualization without adversely impacting the ability of the guide catheter1340 to minimally impact heart features.

In various other embodiments, an ICE Sensor array 1342 may be disposed at other locations proximate to the distal end of the catheter system. For example, sensor array 1342 may be disposed on a surface of the tissue engagement mechanisms 735 of working catheter 730 as shown in FIG. 13B, to provide a visual range 1350 enabling a user to visualize the tissue engagement mechanisms 735 as well as the distal end of guide catheter 910. Alternatively, sensor array 1342 may be disposed at the distal end of the working catheter 730 as shown in FIG. 13C, to provide the visualization range 1352 enabling visualization of the distal end of the working catheter 910 as well as the tissue engagement mechanisms 735.

Alternative embodiments may deploy an ultrasound catheter, such as an Acuson IPX8 AcuNav catheter within a central lumen of the working catheter as disclosed in U.S. Patent 10,555,813, entitled IMPLANTABLE DEVICE AND DELIVERY SYSTEM FOR RESHAPING A HEART VALVE ANNULUS, and incorporated herein by reference in its entirety. Using either method, by rotating the ultrasound device around the inside of the valve annulus, the relative position of the implant components and valve leaflets may be more easily visualized for increased accuracy in the annuloplasty procedure.

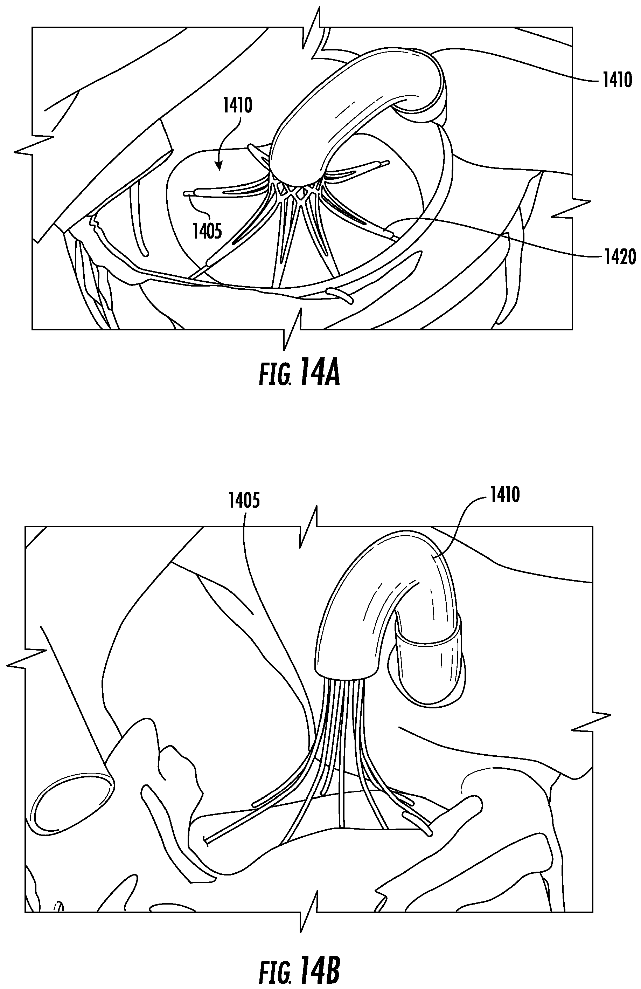

As discussed above, the concepts disclosed herein which include using a guide catheter to more precisely target treatment sites may be adapted for use with a variety of annuloplasty systems. FIGS. 14A and 14B illustrate the adaptation of the described techniques with a frame-based annuloplasty procedure. As shown in FIG. 14A, the introducer sheath 1410 may be introduced above a valve 1450, and a plurality of guidewires, such as guidewire 1405, may be advanced to positions proximate to the valve 1450 using a stent like frame 1420 as a tissue engaging member. Distal guidewire anchors may be driven into the tissue proximate to the valve 1450, and as shown in FIG. 14B, the frame may subsequently be removed. Guidewires 1405 may remain during the annuloplasty procedure to direct annuloplasty components to treatment sites around the valve. The guidewires 1405 may be removed once treatment is completed.

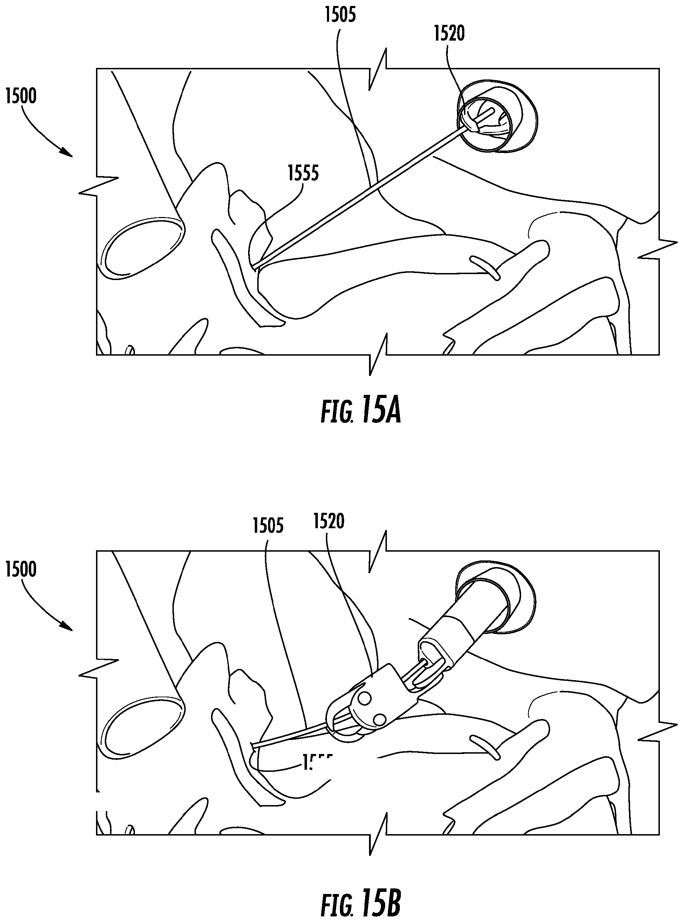

FIGS. 15A and 15B illustrate an alternate embodiment of an annuloplasty system 1500 which uses a guide catheter 1505 as disclosed herein to direct an annuloplasty "Over-the-Wire" (OTW) tool 1520 to a treatment site 1555. As shown in FIG. 15A, the guide catheter 1505 may first be directed to a treatment site 1555 in the cardiac wall. As shown in FIG. 15B, the OTW tool 1520 may then be precisely directed over the guide catheter 1505 to the treatment site 1555, increasing the precision of treatment while decreasing the potential for inadvertent contact. Accordingly, the principles disclosed herein provide a straightforward, low-impact, highly flexible solution to valve repair and replacement.

Thus, the disclosure is not intended to be limited to the implementations shown herein but is to be accorded the widest scope consistent with the claims, the principles and the novel features disclosed herein. The word "example" is used exclusively herein to mean "serving as an example, instance, or illustration." Any implementation described herein as "example" is not necessarily to be construed as preferred or advantageous over other implementations, unless otherwise stated.

Certain features that are described in this specification in the context of separate implementations also can be implemented in combination in a single implementation. Conversely, various features that are described in the context of a single implementation also can be implemented in multiple implementations separately or in any suitable sub-combination. Moreover, although features can be described above as acting in certain combinations and even initially claimed as such, one or more features from a claimed combination can in some cases be excised from the combination, and the claimed combination can be directed to a sub-combination or variation of a sub-combination. Similarly, while operations are depicted in the drawings in a particular order, this should not be understood as requiring that such operations be performed in the particular order shown or in sequential order, or that all illustrated operations be performed, to achieve desirable results. Additionally, other implementations are within the scope of the following claims. In some cases, the actions recited in the claims can be performed in a different order and still achieve desirable results.

It will be understood by those within the art that, in general, terms used herein are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations. In addition, even if a specific number of an introduced claim recitation is explicitly recited, those skilled in the art will recognize that such recitation should typically be interpreted to mean at least the recited number (e.g., the bare recitation of "two recitations," without other modifiers, typically means at least two recitations, or two or more recitations). Furthermore, in those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., "a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

The devices and/or methods disclosed and claimed herein can be made and executed without undue experimentation in light of the present disclosure. While various embodiments of the devices and methods of this disclosure have been described, it may be apparent to those of skill in the art that variations can be applied to the devices and/or methods and in the steps or in the sequence of steps of the method described herein without departing from the concept, spirit and scope of the disclosure. All such similar substitutes and modifications apparent to those skilled in the art are deemed to be within the spirit, scope and concept of the disclosure as defined by the appended claims.

### EMBODIMENTS

Although the present invention is defined in the attached claims, it should be understood that the present invention can also (alternatively) be defined in accordance with the following embodiments:
1. A system comprising:
   an introducer sheath having a lumen extending from a proximal end to a distal end and configured to be transluminally advanced into a cardiac cavity including a treatment site;
   a working catheter configured for longitudinal translation within the introducer sheath to position a distal tip of the working catheter proximate to the distal end of the introducer sheath; and
   a guide catheter translationally disposed within the working catheter, the guide catheter comprising a translation mechanism configured to advance a distal end of the guide catheter through the distal end of the working catheter to the treatment site to provide a path to the treatment site for the working catheter.
2. The system of embodiment 1, wherein the guide catheter comprises a distal guidewire anchor disposed on the distal end, and the translation mechanism is configured to drive the distal guidewire anchor into tissue of the treatment site to secure the guide catheter to the treatment site.
3. The system of embodiment 2, wherein the distal guidewire anchor comprises at least one of a helical member, a clamp member, a hook member, or a needle member.
4. The system of embodiments 1 or 3, wherein a proximal end of the introducer sheath further comprises a steering controller coupled to the distal end of the introducer sheath to guide the distal end of the introducer sheath to the cardiac cavity.
5. The system of embodiment 3, wherein the working catheter is an annuloplasty catheter including a tissue engagement mechanism at its distal end.
6. The system of embodiment 5, wherein the tissue engagement mechanism includes at least one of a needle, a hook, a clamp, a screw, a frame or an anchor.
7. The system of embodiment 5, wherein the working catheter comprises a tissue engagement control mechanism coupled to control the tissue engagement mechanism.
8. The system of embodiment 5, further comprising an implant stylet, slideably disposable within a working channel of the working catheter, the implant stylet comprising at least one implant component supported within a distal end of the implant stylet.
9. The system of embodiment 8, wherein the implant stylet comprises at least one implant release mechanism disposed at proximal end of the implant stylet, the at least one implant release mechanism configured to release at least one implant component from the implant stylet.
10. The system of embodiments 1 or 8, further comprising a staging structure comprising at least one of an introducer sheath support, a working catheter support or an implant stylet support.
11. The system of embodiment 10, wherein the staging structure comprises a catheter stand comprising a rail, and wherein the working catheter support is slideably disposed on the rail of the catheter stand such that movement of the working catheter support along the rail translates the working catheter within the introducer sheath.
12. The system of embodiments 1 or 8, wherein the translation mechanism is further configured to retract the guide catheter into the distal end of the working catheter.
13. The system of embodiments 1 or 8, comprising a visualization mechanism disposed proximate to the distal guidewire anchor.
14. The system of embodiment 13, wherein the guide catheter comprises the visualization mechanism.
15. The system of embodiment 13, wherein the visualization mechanism comprises an Intra-Cardiac Echography (ICE) transducer.
16. A system comprising: an introducer sheath having a lumen extending from a proximal end to a distal end and configured to be transluminally advanced into a cardiac cavity including a treatment site; a working catheter configured for longitudinal translation within the introducer sheath to position a distal end of the working catheter proximate to the distal end of the introducer sheath; and a guide catheter translationally disposed within the working catheter, the guide catheter comprising a translation mechanism configured to advance a distal end of the guide catheter through the distal end of the working catheter to the treatment site to provide a path to the treatment site for the working catheter.
17. The system of embodiment 16 wherein the guide catheter comprises a distal guidewire anchor disposed at the distal end and the translation mechanism is configured to drive the distal guidewire anchor into tissue of the treatment site to secure the guide catheter to the treatment site.
18. The system of embodiment 17 wherein the distal guidewire anchor comprises at least one of a helical member, a clamp member, a hook member, or a needle member.
19. The system of embodiment 18 wherein a proximal end of the introducer sheath further comprises a steering controller coupled to the distal end ofthe introducer sheath to guide the distal end of the introducer sheath to the cardiac cavity.
20. The system of embodiment 18 wherein the working catheter comprises a distal tissue engagement mechanism and a proximal tissue engagement control mechanism.
21. The system of embodiment 20 further comprising an implant stylet, slidably disposable within a working channel of the working catheter, the implant stylet comprising at least one implant component supported within a distal end of the implant stylet.
22. The system of embodiment 21 further comprising a staging structure comprising at least one of an introducer sheath support, a working catheter support or an implant stylet support.
23. The system of embodiment 22 wherein the staging structure comprises a catheter stand comprising a rail, and wherein the working catheter support is slideably disposed on the rail of the catheter stand such that movement of the working catheter support along the rail translates the working catheter within the introducer sheath.
24. The system of embodiment 18 wherein the translation mechanism is further configured to retract the guide catheter into the distal end of the working catheter.
25. The system of embodiment 17 comprising a visualization mechanism disposed proximate to the distal guidewire anchor upon one of the guide catheter, the distal guidewire anchor or the working catheter.
26. The system of embodiment 25 wherein the visualization mechanism comprises an Intra-Cardiac Echo (ICE) transducer.
27. A method of annuloplasty comprising: transluminally advancing an introducer sheath having a lumen extending from a proximal end to a distal end into a cardiac cavity including a treatment site; advancing a distal end of a working catheter to the distal end of the introducer sheath; advancing a guide catheter past the distal end of the working catheter to the treatment site to provide a path for the working catheter, and anchoring the distal end of the guide catheter to the treatment site.
28. The method of embodiment 27 further comprising translating the working catheter to the treatment site along the path provided by the guide catheter.
29. The method of embodiment 28 further comprising withdrawing the working catheter from the treatments site over the guide catheter to position the distal end of the working catheter proximate to the distal end of the introducer sheath.
30. The method of embodiment 29 further comprising releasing the distal end of the guide catheter from the treatment site, advancing the distal end of the guide catheter to a second treatment site, and advancing the distal end of the working catheter to the second treatment site over the guide catheter.
31. A catheter system comprising: a working catheter configured for advancement within an introduction catheter to at least a position within a cardiac cavity where a distal tip of the working catheter is removed from a treatment site of the cardiac cavity; a guide catheter translationally disposed within the working catheter, the guide catheter comprising a translation mechanism configured to advance the guide catheter through the working catheter to the treatment site, the guide catheter comprising an attachment mechanism for coupling a distal end of the guide catheter to the treatment site; and a mechanism configured to slidably advance the working catheter along the guide catheter to the treatment site.
32. The catheter system of embodiment 31 further including a distal guidewire anchor coupled to the guide catheter and the translation mechanism is configured to drive the distal guidewire anchor into tissue of the treatment site to secure the guide catheter to the treatment site.
33. The catheter system of embodiment 32 wherein the distal guidewire anchor comprises at least one of a helical member, a clamp member, a hook member, or a needle member.
34. The catheter system of embodiment 33 wherein the working catheter is an annuloplasty catheter including a tissue engagement mechanism at its distal end including at least one of a needle, a hook, a clamp, a screw, a frame or an anchor.
35. The catheter system of embodiment 31 further comprising a visualization mechanism disposed proximate to the attachment mechanism ofthe guide catheter.

## Claims

1. A system comprising:
an introducer sheath having a lumen extending from a proximal end to a distal end and configured to be transluminally advanced into a cardiac cavity including a treatment site;
a working catheter configured for longitudinal translation within the introducer sheath to position a distal tip of the working catheter proximate to the distal end of the introducer sheath; and
a guide catheter translationally disposed within the working catheter, the guide catheter comprising a translation mechanism configured to advance a distal end of the guide catheter through the distal end of the working catheter to the treatment site to provide a path to the treatment site for the working catheter.

2. The system of claim 1, wherein the guide catheter is a steerable catheter.

3. The system of claim 1 or claim 2, wherein the working catheter includes a distal end, a proximal end and a working shaft disposed therebetween, wherein the proximal end includes a handle comprising a guide catheter control portion.

4. The system of claim 3, wherein the guide catheter control portion includes a proximal end of the guide catheter, disposed within a channel of the working shaft, and a guide catheter lock.

5. The system of claim 3 or claim 4, wherein the guide catheter control portion includes a steering controller positioned on a first surface and the translation mechanism disposed on a different surface thereof.

6. The system of claim 4 or claim 5, when dependent on claim 4, wherein the guide catheter lock comprises a first position in which the guide catheter lock enables axial translation of the guide catheter within the channel and steering of the guide catheter, and a second position in which the guide catheter lock locks the distal end of the guide catheter in a fixed position.

7. The system of any one of claims 3 to 6, wherein the handle comprises a tissue engagement control portion including controls configured to steer a distal end of the working shaft.

8. The system of claim 7, wherein the tissue engagement control portion comprises a tissue engagement lock having a first position in which the tissue engagement lock enables steering of the distal end of the working shaft, and a second position in which the tissue engagement lock locks the distal end of the working shaft in a fixed position.

9. The system of any one of the preceding claims, wherein a proximal end of the introducer sheath further comprises a steering controller coupled to the distal end of the introducer sheath to guide the distal end of the introducer sheath to the cardiac cavity.

10. The system of any one of claims 7 to 9, when dependent on claim 7, wherein the working catheter includes a tissue engagement mechanism at its distal end, wherein the tissue engagement control portion is coupled to the tissue engagement mechanism to control the tissue engagement mechanism.

11. The system of any one of the preceding claims, further comprising a staging structure comprising at least one of an introducer sheath support, a working catheter support or an implant stylet support.

12. The system of claim 11, wherein the staging structure comprises a catheter stand comprising a rail, and wherein the working catheter support is slideably disposed on the rail of the catheter stand such that movement of the working catheter support along the rail translates the working catheter within the introducer sheath.

13. The system of claim 12, when dependent on claims 3 and 7, wherein the catheter stand comprises a first bracket configured to support the tissue engagement control portion and a second bracket configured to support the guide catheter portion.

14. The system of claim 12 or claim 13, wherein the introducer sheath comprises a shaft coupled to a handle at the proximal end of the introducer sheath, wherein the handle comprises a feature configured to engage the handle to the catheter stand.

15. The system of any one of the preceding claims, further comprising an implant stylet, slideably disposable within a working channel of the working catheter, the implant stylet comprising at least one implant component supported within a distal end of the implant stylet.
